# EUROPEAN PATENT APPLICATION

(11) **EP 1 447 450 A1**
(43) Date of publication of application: **18.08.2004**
(21) Application number: 02768117.0
(22) Date of filing: 27.09.2002
(51) Int. Cl.: C12N 15/86, A61K 48/00

(54) **METHOD OF REGULATING EXPRESSION OF GENE CARRIED ON VIRAL VECTOR**

(30) Priority: 27.09.2001 JP 2001298223
(71) Applicant: Dnavec Research Inc., Tsukuba-shi, Ibaraki 305-0856 (JP)
(72) Inventor: INOUE, Makoto, Tsukuba-shi, Ibaraki 305-0856 (JP); IIDA, Akihiro, Tsukuba-shi, Ibaraki 305-0856 (JP); HASEGAWA, Mamoru, Tsukuba-shi, Ibaraki 305-0856 (JP)
(74) Representative: Warcoin, Jacques
(86) International application number: PCT/JP2002/010065
(87) International publication number: WO 2003/029474

(57) **Abstract**

The present inventors revealed that non-steroidal anti-inflammatory drugs and muscle relaxants suppressed the expression of genes carried on viral vectors. These agents also suppressed viral vector cytotoxicity. The present invention provides a method for regulating the expression of genes carried on viruses by using non-steroidal anti-inflammatory drugs and/or muscle relaxants. The effect of these agents is reversible, and viral vector gene expression and cytotoxicity increased after termination of agent administration. The agents of the present invention are useful for regulating the expression of viral and therapeutic genes, and for suppressing viral vector cytotoxicity in gene therapy using viral vectors.

## Description

### Technical Field

The present invention relates to method for regulating the expression of genes carried on a viral vector by using non-steroidal anti-inflammatory drugs and such.

### Background Art

Viral vectors are being used in many gene transfer methods, including gene therapy, because they are highly efficient in transferring genes into cells, and thus facilitate a high expression of the transferred gene. However, methods for regulating the expression of genes transferred into cells by viral vectors are virtually unknown. The development of a method for regulating the expression of genes carried on viral vectors, or for reducing viral cytotoxicity, is anticipated.

Chen *et al*. reported that aspirin suppressed the multiplication of the Vesicular Stomatitis virus (VSV) (Chen, N. *et al*., Virology 276, 44-51 (2000)). However, Chen *et al*. did not disclose the effect of aspirin on the expression of genes carried on the VSV virus. Equally, Chen *et al*. did not suggest the use of aspirin to regulate the expression of genes carried on vectors for use in gene transfer, nor did they examine whether it was possible to reversibly regulate viral multiplication when aspirin was removed.

In recent years, viral gene manipulation has advanced the development of multiplicative viral vectors which can multiply in tumor cells, but are not pathogenic in normal tissue. Of these multiplicative viruses, Herpes Simplex Virus (HSV), Adeno Virus, and Reo Virus are drawing attention. Immuno gene therapy and suicide gene therapy are attracting attention as methods for increasing the effect of multiplicative viruses as gene expression vectors. The use of tissue specific promoters to enhance the specificity of multiplicative vectors is also being investigated (Fujidou, N., Gene diagnosis and therapy for brain tumors, Front line gene therapy research, Brain tumor treatment using multipliable viral vectors, Nounokagaku (Brain Science), Vol. 23, No. 5, 379-387, 2001). As a vector, HSV is characteristic in that it can transiently express target genes, and can be transfected into non-dividing cells, such as nerve cells. The HSV vector is drawing attention because it enables the following : 1) the use of HSV's ability to damage cells; 2) the use of a hepatoma cell-specific modified HSV strain which attacks certain tumor cells; 3) the use of a deleted-HSV strain which uses an amplicon plasmid and helper HSV (Miyatake, S., Development and prospects of viral vectors used for gene therapy, Herpes Simplex Virus as a vector for gene therapy, Virus, Vol. 47, No. 2 239-246, 1997).

An advantage of viral vectors such as the Herpes Simplex virus, Adeno virus, and Reo virus is that they can be actively utilized in cytotoxic therapies. However, these vectors cannot be produced without cells, and therefore, their advantage becomes a disadvantage when producing large quantities for medical application as cultured cells can not provide sufficient quantities of these viruses. If a drug for regulating cytotoxicity can be used during cell culture, the addition of the drug is expected to yield the virus.

### Disclosure of the Invention

The present invention provides a method for regulating the expression of genes carried on viral vectors using non-steroidal anti-inflammatory drugs (NSAIDs) and/or muscle relaxants. The present invention also provides agents that regulate the expression of genes carried on viral vectors. The present inventors diligently studied the effect of applying various drugs to mammals, including humans, on the expression of genes carried on viral vectors. Surprisingly, the results of this research revealed that NSAIDs and muscle relaxants had the activity of significantly suppressing the expression of genes carried on viral vectors. In cells transfected with a viral vector, it was revealed that the expression of genes carried on this vector was significantly suppressed by all of the NSAIDs tested, including aspirin, ketoprofen, flurbiprofen, indomethacin, diclofenac, and mefenamic acid. Papaverin, a smooth muscle relaxant, also strongly suppressed the expression of genes carried on viral vectors.

The effect of these agents on cytotoxicity caused by vector transfer was examined in cells transfected with viral vectors. The results showed that aspirin, ketoprofen, flurbiprofen, and indomethacin were extremely effective in reducing cell damage in these cells. Of the above drugs, aspirin and ketoprofen were especially effective in suppressing cytotoxicity.

Detailed studies of the suppression of expression of genes carried on viral vectors revealed that suppression by aspirin was temporary, and disappeared a few days after suspension of drug administration. This indicates that administration of an agent of the present invention can reversibly regulate the expression of genes carried on viral vectors, or regulate viral vector cytotoxicity. Thus, when regulation is no longer necessary, administration of an agent of the present invention can be ceased, and regulation of viral vector expression can be reversed. Expression of genes carried by the viral vector can be restored by suspending administration of the agent as required. Therefore, dynamic regulation of the expression of genes carried on viral vectors can be achieved by the present invention.

Drugs of the present invention can rapidly regulate the expression level and cytotoxicity of a gene carried on a viral vector, such as a therapeutic gene transferred by a viral vector, in a clinical setting, for example, in gene therapy. The present invention relates to a method for regulating the expression of genes carried on viral vectors using non-steroidal anti-inflammatory drugs (NSAIDs) and/or muscle relaxants, and also relates to agents that regulate expression of genes carried on viral vectors. More specifically, the present invention relates to:
(1) a method for suppressing the expression of a gene carried on a viral vector, including the step of contacting a non-steroidal anti-inflammatory drug and/or a muscle relaxant with cells transfected by the viral vector;
(2) the method according to (1), in which cytotoxicity of the viral vector is reduced;
(3) the method according to (1), in which the non-steroidal anti-inflammatory drug is selected from the group consisting of aspirin, ketoprofen, fulurbiprofen, indomethacin, diclofenac, and mefenamic acid;
(4) the method according to (3), in which the non-steroidal anti-inflammatory drug is selected from the group consisting of aspirin, ketoprofen, flurbiprofen, and indomethacin;
(5) the method according to (4), in which the non-steroidal drug is aspirin and/or ketoprofen;
(6) the method according to (1), in which the muscle relaxant is papaverin;
(7) the method according to any one of (1) to (6), in which the viral vector is a minus-strand RNA viral vector;
(8) the method according to (7), in which the minus-strand RNA virus is Paramyxovirus;
(9) the method according to (8), in which the Paramyxovirus is Sendai virus;
(10) an agent for suppressing the expression of a gene carried on a viral vector, wherein the agent comprises a non-steroidal anti-inflammatory drug and/or a muscle relaxant; and
(11) the agent according to (10), in which the agent has the ability to reduce the cytotoxicity of the viral vector.

The present invention provides a method for regulating the expression of a gene carried on a viral vector. In particular, the method of the present invention includes the step of contacting an NSAID and/or a muscle relaxant with cells transfected by a viral vector. The NSAID and/or muscle relaxant is not restricted, and a desired NSAID and/or muscle relaxant can be used alone or in multiple combinations.

The expression "non-steroidal anti-inflammatory drugs" (NSAIDs) is a generic term referring to a group of drugs that, in addition to steroids, have an anti-inflammatory effect and which are also called "aspirin-like drugs" (Goodman & Gilman's The Pharmacological Basis of Therapeutics, p. 775). In particular, acidic NSAIDs are known to suppress prostaglandin biosynthesis (Vane, J. R. and Botting, R., FASEB J. 1, 89-96 (1987); Vane, J. R., Nat. New Biol. 231 (25), 232-235 (1971)). NSAIDs include, for example, salicilic acid-type (aspirin salts such as salicilic acid, aspirin, and aspirin DL lysine, disulfonyl), allyl acetic acid-type (indomethacin, diclofenac, sulindac, nabumetone, progulumethacin, indomethacinfarnesyl, and etodolac), propionic acid-type (ibuprofen, naproxen, flurbiprofen, tiaprofen, pranoprofen, loxoprofen, aluminoprofen, and ketoprofen), fenamic acid-type (mefenamic acid, tolufenamic acid), pyrazolone-type (phenylbutazone, oxifenbutazone), oxicam-type (piroxicam, tenoxicam, and ampiroxicam). Non-acidic NSAIDs include, but are not limited to, epirizole, tiaramid, and emorfazone. The NSAIDs used in the present invention are preferably acidic NSAIDs, and are more preferably NSAIDs including aspirin, ketoprofen, flurbiprofen, indomethacin, diclofenac (including diclofenac sodium), and mefenamic acid; even more preferably, NSAIDs including aspirin, ketoprofen, flurbiprofen, and indomethacin; and especially preferably, NSAIDs including aspirin and ketoprofen.

Muscle relaxants used in the present invention include, but are not limited to, for example, skeletal muscle relaxants (tubocurarine, suxamethonium, decamethonium, and dantrolene) and smooth muscle relaxants (papaverin, ritodrne, terbutaline, and magnesium sulfate). The muscle relaxants preferably used in the present invention include smooth muscle relaxants. In particular, opium alkaloids, which function as smooth muscle relaxants, and preferably isoquinolin derivatives such as papaverine, are included.

The above-mentioned NSAIDs and/or muscle relaxants can also take the form of a salt (for example, a sodium salt), hydrated material, complex, or such. Additionally, isoforms, analogs, derivatives, and esters thereof are also included. Agents in the present invention are useful as reagents and pharmaceuticals, or as raw materials for pharmaceuticals to suppress the expression of a gene carried on a viral vector, in cells transfected by that viral vector. These agents are also useful as reagents and pharmaceuticals, or as raw materials for pharmaceuticals to reduce cytotoxicity of the viral vector.

In a method of the present invention, NSAIDs and/or muscle relaxants are contacted with cells transfected with a viral vector, in order to suppress the expression in cells of genes carried on that viral vector. The contact can be carried out prior to, at the same time as, or after the transfection of cells with the viral vector. All of these embodiments are included in the present invention. The expression of genes carried on a viral vector, in cells transfected with that viral vector, is suppressed by contacting the an agent of the present invention with these cells.

In the present invention, "genes carried on a viral vector" indicates genes transferred into cells by that vector. These genes include all genes transferred into cells, such as foreign genes inserted into the vector to be expressed in cells, viral genes originating from the viral vector, and marker genes possibly carried by the vector. Moreover, the term "gene" indicates genetic material and includes nucleic acids such as RNA and DNA. Genes may or may not code proteins, and may, for example, code a functional RNA such as a ribozyme or anti-sense RNA. Genes can be derived from either a natural sequence or an artificially constructed sequence. In the present invention the term "DNA" includes both single-stranded and double-stranded DNA.

The methods of the present invention can be suitably applied to regulate the expression of foreign genes encoded in viral vectors in particular. For example, the methods of the present invention can be used to suppress foreign gene expression in cells transfected with a viral vector which expresses foreign genes. For example, using a method of the present invention, the expression of therapeutic genes can be suppressed in gene therapy using viral vectors, where high expression of therapeutic genes causes undesirable side effects. The foreign gene is not restricted and includes, for example, genes coding for biologically active proteins which may be functional in host cells after vector transfer, and especially, cytokines/chemokines, hormones, enzymes, soluble receptors, and antibody fragments.

The methods of the present invention can also suppress the expression of viral genes possessed by viral vectors. Many of the proteins that are expressed from viral genes are known to induce cell damage in transfected cells when expressed. It is possible to reduce viral vector cytotoxicity by suppressing the expression of genes possessed these vectors, using the methods of the present invention. As mentioned above, viral vectors based on the Herpes Simplex virus, Adeno virus, and Reo virus are all cytotoxic. The present invention is useful in suppressing cell damage caused by these viral vectors.

The methods of the present invention can be used to suppress the expression of genes carried on desired viral vectors. Viral vectors that can infect mammalian cells are preferred in the application of the present invention. These vectors include, for example, Retroviral vectors, Lentiviral vectors, Adenoviral vectors, and Adeno-associated viral vectors. Preferably, they include Herpes Simplex virus vectors, Adenoviral vectors, Retroviral vectors, Semliki Forest viral vectors, Sindbis viral vectors, Vaccinia virus vectors, and Fowlpox virus vectors. Most preferably, they include but are not limited to minus-strand RNA viral vectors such as Rabies virus vectors, Measles virus vectors and Sendai virus vectors. Moreover, as mentioned above, cytotoxic viral vectors are suitable for use in the present invention. The present invention provides methods to reduce cell damage caused by viral vectors, including the step of contacting NSAIDs and/or muscle relaxants with cells transfected by viral vectors. Agents including NSAIDs and/or muscle relaxants can be used as agents to reduce cytotoxicity caused by viral vectors.

In the present invention, a "minus-strand RNA virus" is an RNA virus whose genome comprises a minus (negative) strand. Minusstrand RNA viruses include, for example, Sendai virus, Newcastle disease virus, Mumps virus, Measles virus, Respiratory syncytial virus, Rinderpest virus and Distemper virus in the Paramyxovirus family (*Paramyxoviridae*); Influenza virus in the Orthmyxovirus family (*Orthomyxoviridae*); and Vesicular stomatitis virus and Rabies virus in the Rhabdovirus family (*Rhabdoviridae*). Of the viruses mentioned above, the preferred minus-strand RNA viral vector in the present invention is a non-segmented (that is, single stranded) minus-strand RNA virus, and more preferably, it is paramyxovirus.

Herein, a "paramyxovirus" is defined as a virus belonging to the Paramyxovirus family (*Paramyxoviridae*), or a derivative thereof. Paramyxovirus is a virus group which comprises a non-segmented minus-strand RNA as a genome, and includes the Paramyxovirus sub-family (*Paramyxovirinae*) (including Genus Respirovirus (also called Genus Paramyxovirus), Genus Rubravirus, and Genus Morbillivirus) and the Pneumovirus sub-family (Pneumovirinae) (including Genus Pneumovirus and Metapneumovirus). Paramyxoviruses applicable in the present invention include, for example, Sendai virus, Newcastle disease virus, Mumps virus, Measles virus, Respiratory syncytial virus, Rinderpest virus, Distemper virus, Simian Parainfluenza virus (SV5), and Types 1, 2, and 3, Human Parainfluenza virus. Paramyxoviruses in the present invention are, preferably, viruses belonging to *Paramyxovirinae* (Genus Respirovirus, Genus Rubravirus, and Genus Morbillivirus), more preferably, belonging to Genus Respirovirus (also called Genus Paramyxovirus) or their derivatives. Genus Respirovirus viruses especially preferable for application in the present invention are, for example, type 1 Human Parainfluenza virus (HPIV-1), type 3 Human Parainfluenza virus (HPIV-3), type 3 Bovine Parainfluenza virus (BPIV-3), Sendai virus (also known as type 1 Mouse Parainfluenza virus), type 10 Simian Parainfluenza virus (SPIV-10), and such. The most preferable Paramyxovirus for application of the present invention is the Sendai virus. These viruses may be natural strains, wild-type strains, mutant strains, laboratory-passaged strains, artificially constructed strains, and so forth. The method of the present invention can also suppress expression of genes carried on incomplete viruses, such as the DI particle (J. Virol. 68, 8413-8417(1994)).

A "minus-strand RNA viral vector" in the present invention indicates a vector which is derived from a minus-strand RNA virus and transfers genes into host cells. A vector is a carrier used to transfer genes into cells. Using the methods of the present invention, the expression of genes carried on both replication competent and replication deficient minus-strand RNA viral vectors can be suppressed. The methods of the present invention can be used to suppress viral vector replication in cells transfected with a replication competent minus-strand RNA viral vector in particular. Therefore, excessive multiplication and transmission of the viral vector can be inhibited.

According to the methods of the present invention, the expression of genes carried on viral vectors can be reversibly suppressed. Thus viral vector gene expression and potential cell damage by the viral vector can be suppressed during the period the agents of the present invention are in contact with transfected cells. When application of the agent is ceased, this suppression is lifted. The methods of the present invention can be used both *in vivo* and *in vitro* (including *ex vivo*). For *in vivo* application, an agent of the present invention can be contacted with virus-transfected cells by administration into the body. There is no limitation as to the administration route, as long as the agent can contact the virus-transfected cells. For example, depending on the characteristics of the active ingredient, administration can be oral, percutaneous, intranasal, transbronchial, intramuscular, intraperitoneal, intravenous, intraarticular, or subcutaneous. For in vitro (including ex vivo) application, an agent of the present invention is added so that it can contact target cells. For example, the agent can be added to cell media.

The concentration of the agent applied is not particularly limited and can be properly determined based on the type of agent and salt, the administration route, and so on. Generally, an agent's effect in suppressing the expression of genes carried on a viral vector is expected to be dependent on the amount of the agent. High doses of some agents might result in cell toxicity, however those skilled in the art can adjust the concentration and administration dose depending on the type of agent and the used purpose. For example, when aspirin is to be used orally, 0.5 to 1.5 g/dose and 1.5 to 4.5 g/day is a standard dose of administration. It is preferable not to exceed the estimated toxicity limit of 300 mg/Kg (1/6 of LD₅₀ 1750 m/kg).

The present invention can be applied to any target individual without limitation, as long as transfection with a viral vector can be performed. For example the target can include mammals and birds, including chickens, quails, mice, rats, dogs, pigs, cats, cows, rabbits, sheep, goats, monkeys, and humans. When *in vitro* (including *ex vivo*) methods are to be used in the present invention, applied targets include, for example, humans, non-human mammals, and birds. When *in vivo* methods are to be used in the present invention, non-human mammals and birds are particularly targeted.

The present invention provides agents that regulate the expression of genes carried on viral vectors. Specifically, the present invention provides agents, including NSAIDs and/or muscle relaxants, to suppress the expression of genes carried on viral vectors. The agents of the present invention include reagents and pharmaceuticals that suppress the expression of genes carried on viral vectors. The agents of the present invention can be NSAIDs and/or muscle relaxants alone, or their compositions combined with sterile water, physiological saline, buffers, salts, stabilizers, preservatives, surfactants, or such. These compositions can be mixed either before use or at the time of use. Agents of the present invention can be formulated according to known pharmaceutical methods. For example, an agent of the present invention may be formulated by suitably combining it with a pharmacologically acceptable carrier or vehicle, specifically, sterile water, physiological saline, vegetable oil, emulsifiers, suspension agents, surfactants, stabilizers, sustained release preparations, and so on. Agents of the present invention can be prepared as a solution, powder, fine granule, granule, tablet, coated tablet, capsule, troche, buccal tablet, elixir, suspension, syrup, nasal drop, inhaler, ointment, patch, suppository, or such. The amount of NSAID and/or muscle relaxant in these formulations can be appropriately determined.

For example, to formulate an oral drug, an excipient, binder, disintegrator, lubricant, coloring agent, or flavoring and fragrance agent can be added as required into one or more types of NSAID and/or muscle relaxant, followed by formulation as a powder, fine granule, granule, tablet, coated tablet and/or capsule.

Examples of excipients include lactose, cornstarch, sucrose, glucose, mannitol, sorbitol, crystalline cellulose, and silicon dioxide. Examples of binders include polyvinyl alcohol, polyvinylether, methylcellulose, ethylcellulose, gum arabic, tragacanth, gelatin, hydroxypropyl methylcellulose, hydroxypropylcellulose, and polyvinylpyroridone. Disintegrators include, for example, cornstarch, agar, gelatin powder, crystalline cellulose, calcium carbonate, sodium bicarbonate, calcium citrate, dextrin, and pectin. Lubricants include, for example, magnesium stearate, talc, polyethylene glycol, silica, and vegetable oil. There are many known coloring, flavoring, and fragrance agents which can be added to pharmaceuticals. These pharmaceuticals can be in the form of a tablet, microcapsule, granule, or, for example, coated with sugar or another material.

Compositions for injection can be formulated according to conventional methods, by mixing the main pharmaceutical with a pH-adjusting agent, solvent or isotonizer, and by mixing with an adjuvant, stabilizer or such as necessary. Raw materials used for formulation may contain raw materials ordinarily used for pharmaceuticals, medicated cosmetics, cosmetics and such. Specifically, the raw materials used include, for example, animal oil, vegetable oil, mineral oil, ester oil, waxes, high-grade alcohols, fatty acids, silicon oil, surfactants, phospholipids, alcohols, polyhydric alcohols, soluble high molecules, clay minerals, and purified water. Furthermore, additives including but not limited to, pH buffers, antioxidants, chelates, antiseptics, fungicide, coloring agents, and flavoring agents may be added as necessary.

The agents of the present invention can be applied using desired administration routes and, for example, can be taken orally, percutaneously, intra-nasally, transbronchially, intramuscularly, intraperitoneally, intravenously, intraarticularly, or subcutaneously, as mentioned above. Moreover, the agents can be administered systemically or locally. The dosage and administration method of agents varies according to the ability of the active ingredient of the pharmaceutical composition to make tissue transitions, the purpose of treatment, and the body weight and age of the subject of administration (e.g. the patient). Proper dosage and administration method can be selected by those skilled in the art. Administration can be conducted once or several times. The agents of the present invention can be applied to desired cultured cells and individuals. The target individuals and cells can be derived from any mammal, including humans, monkeys, mice, rats, rabbits, sheep, cows and dogs, and from other vertebrates. In particular, the agents of the present invention are useful for regulating the expression of genes carried on viruses in human gene therapy using viral vectors. The agents of the present invention are also useful for reversibly suppressing the expression of genes carried on a viral vector. In an especially suitable administration, an agent of the present invention is administrated under the skin, or directly under or in the vicinity of the corneous. The agent is formulated as a percutaneous absorption drug that controls local, percutaneous expression level of a vector. Percutaneous absorption drugs can be in the form of an ointment, cataplasm, tape, etc. Ingredients usually mixed with pharmaceuticals and cosmetics are preferred as bases, adjuvants, or excipients mixed to prepare percutaneous absorption drugs. For example, these drugs can also take the form of a cataplasm whose main component is a polymer which can expand in water, such as a cohesive resin (polyterpene resins, hydrocarbon resins and such), natural or synthetic gum (polyisobutylene, styrene-butylene, styrene-isoprene, styrene-ethylene-butylene, 1,4-polyisoprene and such), or galactomannan. Polyethylene glycols and fatty acid esters can be also combined. Fatty acid esters include, for example, ethyl oleate, isopropyl myristate, isopropyl palmitate, butyl stearate, myristyl myristate, octyldodecyl myristate, cholesteryl stearate, and diisopropyl adipate. Polyethylene glycols include, for example, polyethylene glycol 200, polyethylene glycol 300, polyethylene glycol 400, polyethylene glycol 600, polyethylene glycol 1000, polyethylene glycol 1500, polyethylene glycol 1540, and polyethylene glycol 4000. These combinations may contain absorption polymers as necessary. For example, absorption polymers are preferably able to absorb ten times their weight or more in water, swelling up and becoming a gel. Absorption polymers are preferably micropowders and include carboxy methylcellulose and its metal salts, carboxymethyl polymers and such, in which a small number of cross linking is introduced, polyacrylic acid and its metal salts, sodium salts, carboxymethylated polyvinyl alcohols, or metal salts of saponified starch grafting acrylonitriles.

### Brief Description of the Figures

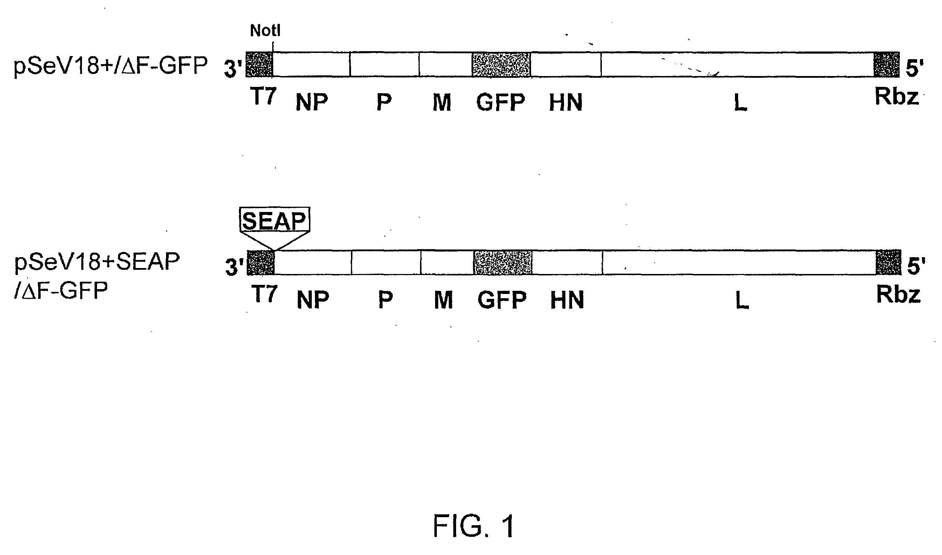

Fig. 1 shows the structure of the SeV gene used to assess agents.

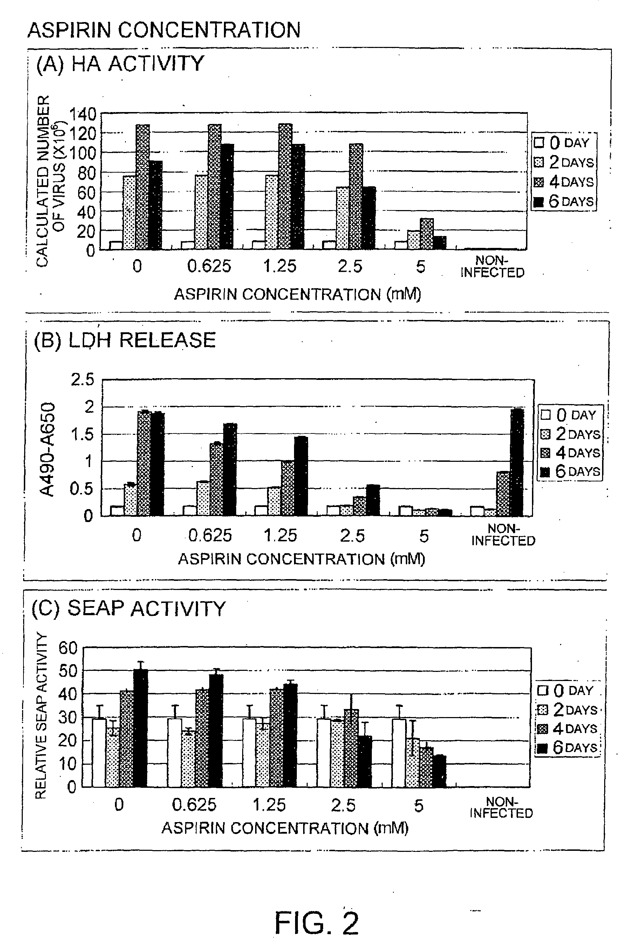

Fig. 2 shows the aspirin concentration-dependent suppression of SeV transcription and replication. LLC-MK2 cells were infected with SeV18+SEAP/ΔF-GFP at MOI=3, two days after infection aspirin was added at the recorded concentrations, and every two days aspirin concentration-dependent suppression of SeV transcription was measured in recovered cultured media. Fig. 2 (A) shows HA activity, which reflects the number of virions. Fig. 2 (B) shows amount of LDH release, which reflects cytotoxicity. Fig. 2 (C) shows time course change of SEAP activity, which reflects the expression of genes carried on the virus.

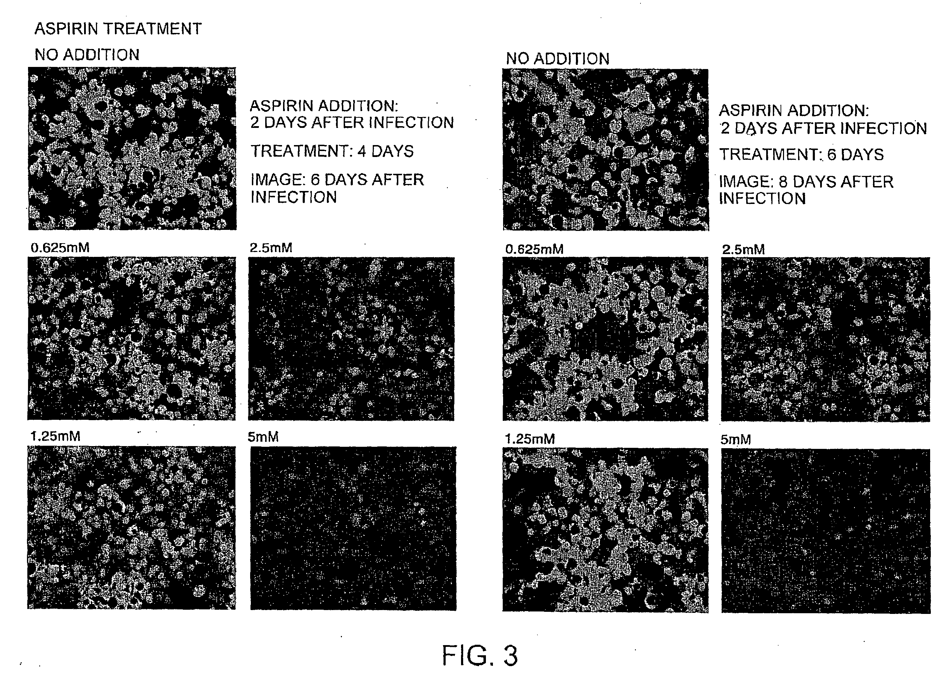

Fig. 3 shows fluorescence microscopy pictures which reflect the amount of GFP expression and cell damage at four and six days after aspirin addition. LLC-MK2 cells were infected with SeV18+SEAP/ΔF-GFP at MOI=3, and aspirin was added two days after infection at the recorded concentrations.

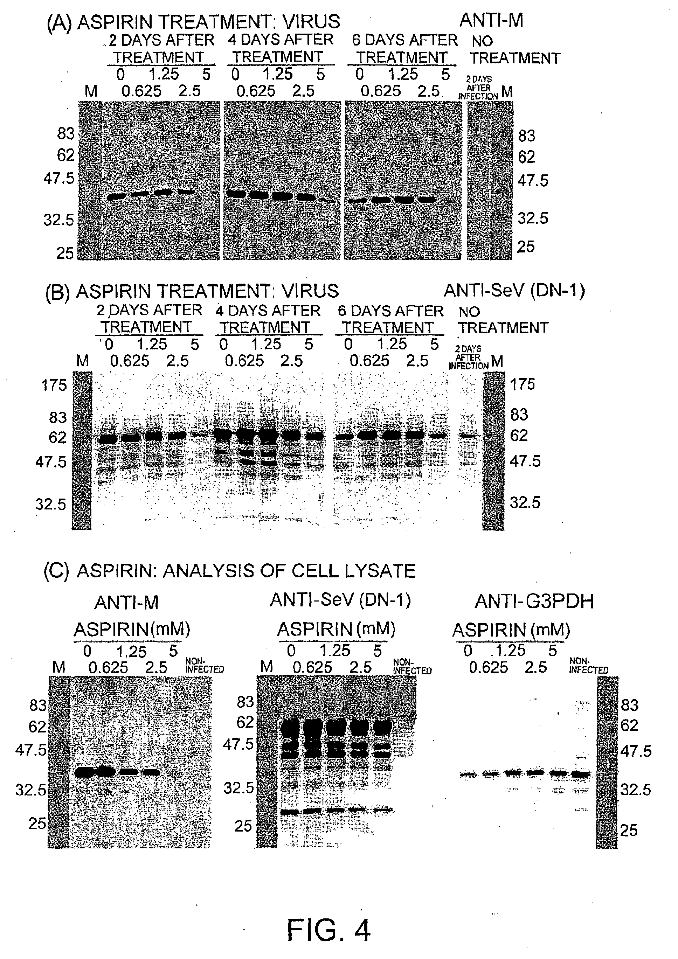

Fig. 4 shows photographs of Western blotting analysis. Analysis was performed by infecting LLC-MK2 cells with SeV18+SEAP/ΔF-GFP at MOI=3, adding aspirin two days after infection, and then centrifuging culture media recovered two, four, and six days after aspirin addition. Western blotting analyses were conducted on the recovered virions using (A) anti-M antibody and (B) DN-1 antibody which mainly recognized NP. Fig 4.(C) shows Western blotting analysis of the cells obtained six days after the addition of aspirin using anti-M antibody, DN-1 antibody, and anti-G3PDH antibody.

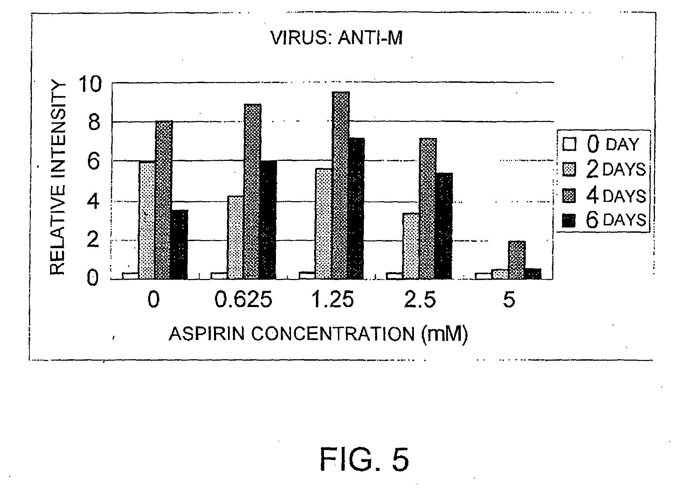

Fig. 5 shows the quantified results of Western blotting using anti-M antibody, conducted on virions recovered after infecting LLC-MK2 cells with SeV18+SEAP/ΔF-GFP at MOI=3, adding aspirin two days after infection, and then centrifuging culture media recovered at two, four, and six days after aspirin addition.

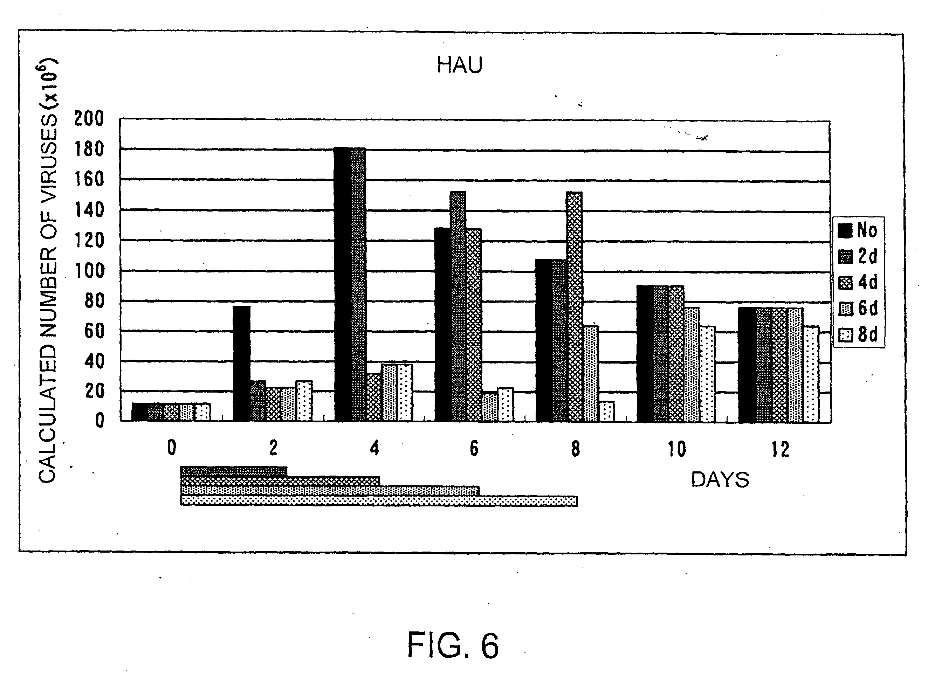

Fig. 6 shows changes in HA activity in recovered culture media obtained by infecting LLC-MK2 cells with SeV18+SEAP/ΔF-GFP at MOI=3, adding 5 mM aspirin two days after infection, and then every two days adding fresh media, either containing 5 mM aspirin, or without aspirin (length of aspirin treatment: 0, 2, 4, 6, and 8 days).

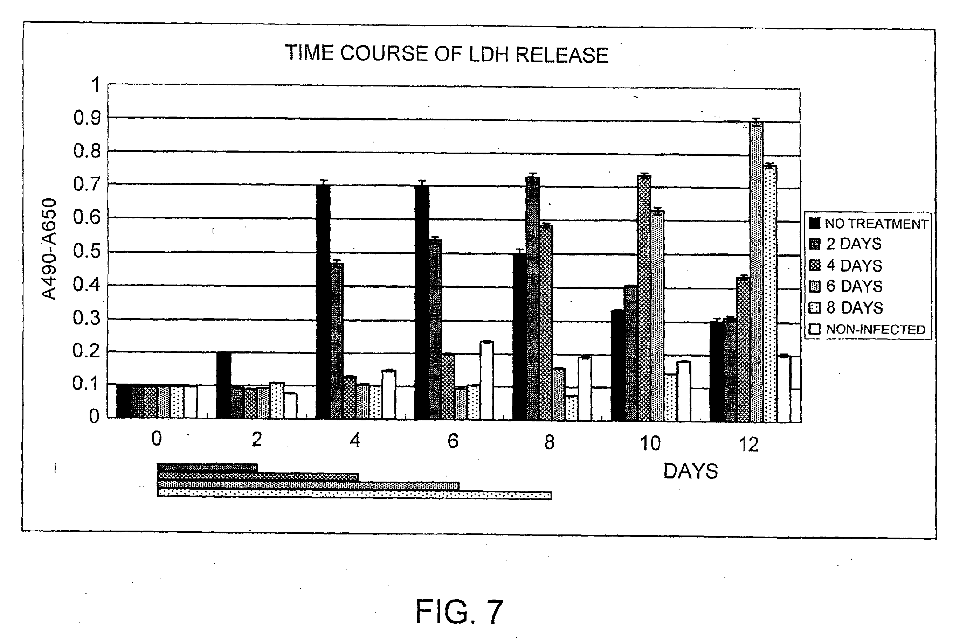

Fig. 7 shows changes in the amount of LDH release in the recovered cultured media obtained by infecting LLC-MK2 cells with SeV18+SEAP/ΔF-GFP at MOI=3, adding 5 mM aspirin two days after infection, and then every two days adding fresh media either containing 5 mM aspirin, or without aspirin (length of aspirin treatment: 0, 2, 4, 6, and 8 days).

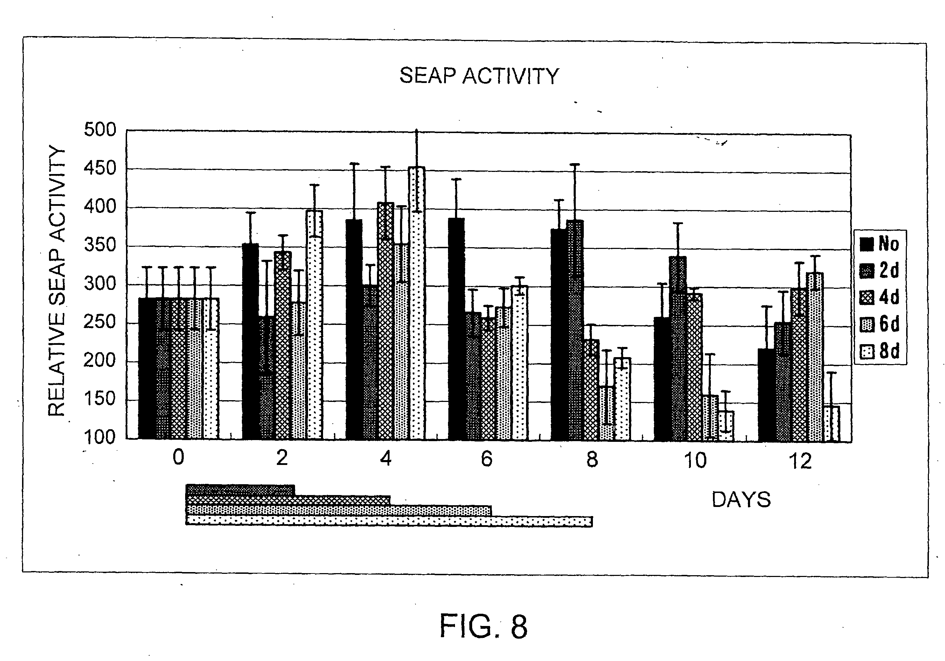

Fig. 8 shows time course changes in SEAP activity in recovered cultured media obtained by infecting LLC-MK2 cells with SeV18+SEAP/ΔF-GFP at MOI=3, adding 5 mM aspirin two days after infection, and then every two days adding fresh media, either containing 5 mM aspirin or without aspirin (length of aspirin treatment: 0, 2, 4, 6, and 8 days).

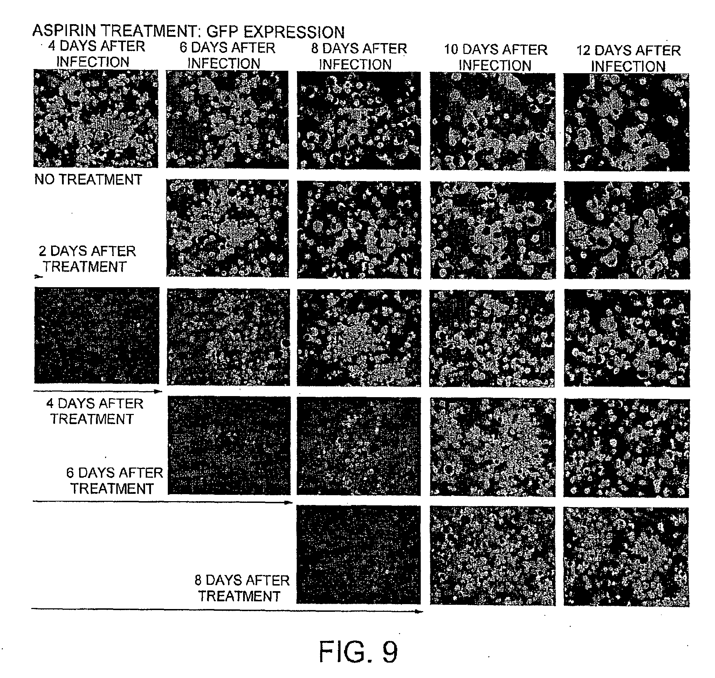

Fig. 9 shows a fluorescence microscopy picture reflecting GFP expression and cell damage, which were observed periodically. LLC-MK2 cells were infected with SeV18+SEAP/ΔF-GFP at MOI=3, 5 mM aspirin was added two days after infection, and then fresh media either containing 5 mM aspirin or without aspirin was added every two days (length of aspirin treatment: 0, 2, 4, 6, and 8 days). The amount of GFP expression and cell damage were periodically observed by fluorescence microscopy.

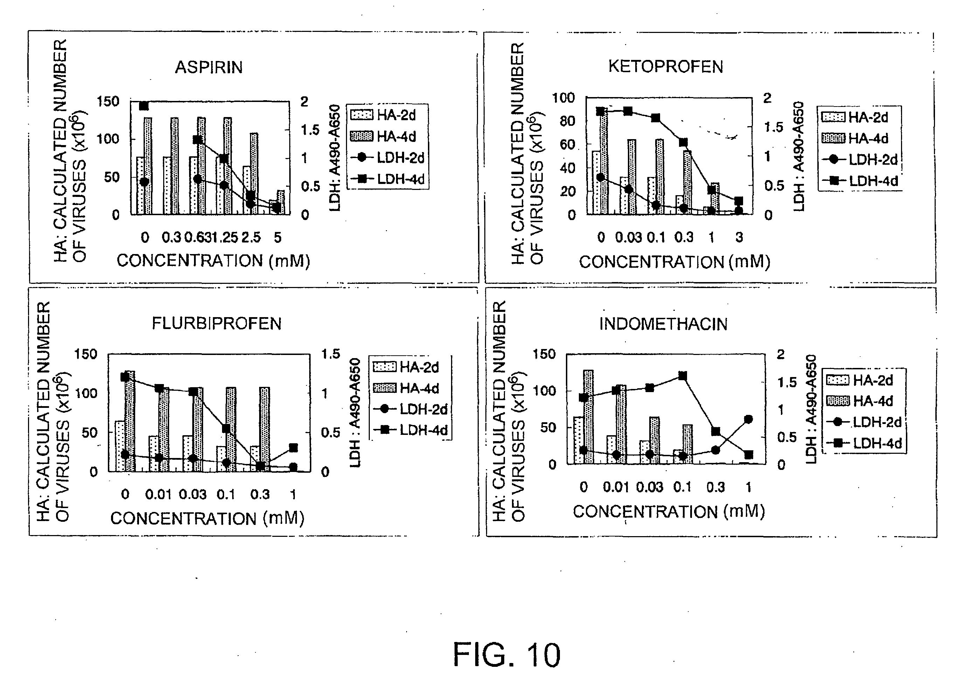

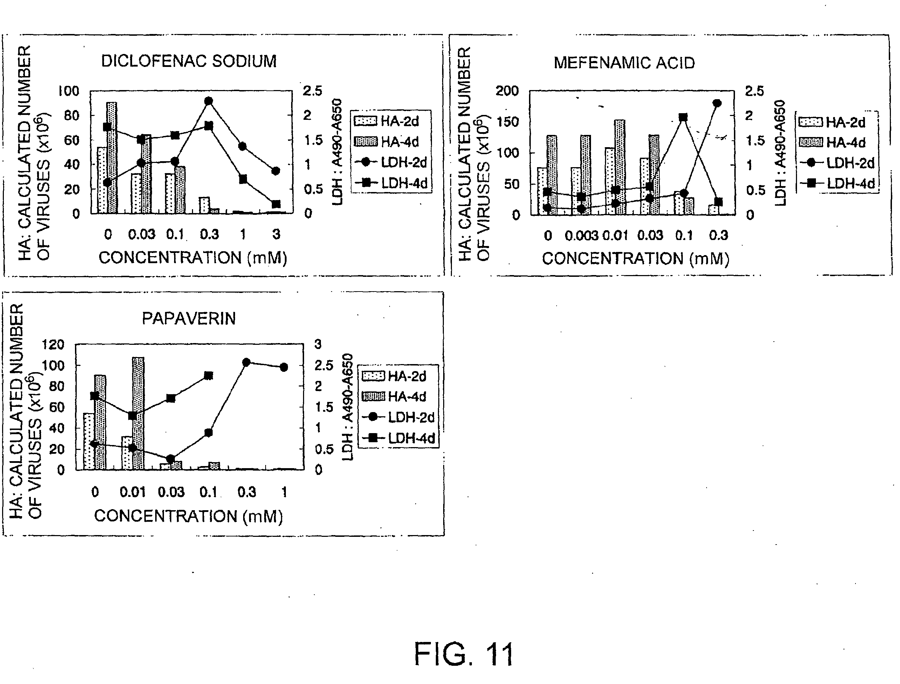

Fig. 10 shows HA activity and the amount of LDH release in the recovered media. LLC-MK2 cells were infected with SeV18+SEAP/ΔF-GFP or SeV18+/ΔF-GFP at MOI=3, and two days after infection, aspirin, ketoprofen, flurbiprofen, indomethacin, diclofenac sodium, mefenamic acid, or papaverine was added at the recorded concentrations. HA activity and LDH release were measured in the recovered cultured media every two days. Fig. 10 continues into Fig. 11.
Fig. 11 shows HA activity and the amount of LDH release in the recovered media. LLC-MK2 cells were infected with SeV18+SEAP/ΔF-GFP or SeV18+/ΔF-GFP at MOI=3, and two days after infection, aspirin, ketoprofen, flurbiprofen, indomethacin, diclofenac sodium, mefenamic acid, or papaverine was added at the recorded concentrations. HA activity and LDH release were measured in the recovered cultured media every two days. Fig. 11 continues from Fig. 10.

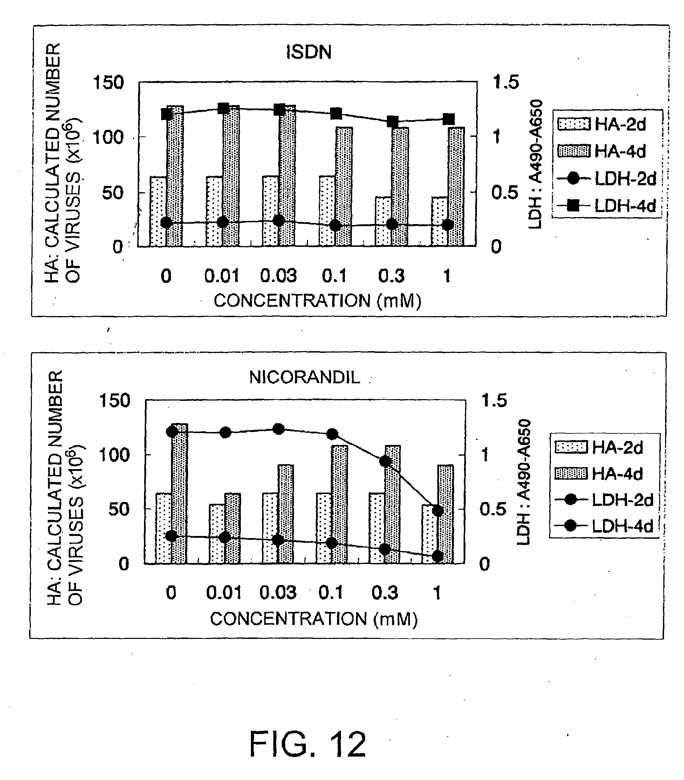

Fig. 12 shows HA activity and the amount of LDH release in the recovered media. LLC-MK2 cells were infected with SeV18+SEAP/ΔF-GFP at MOI=3, and two days after infection, isosorbide dinitrate (ISDN) or nicorandil was added to the media at the recorded concentrations. HA activity and LDH release were measured in the recovered cultured media every two days.

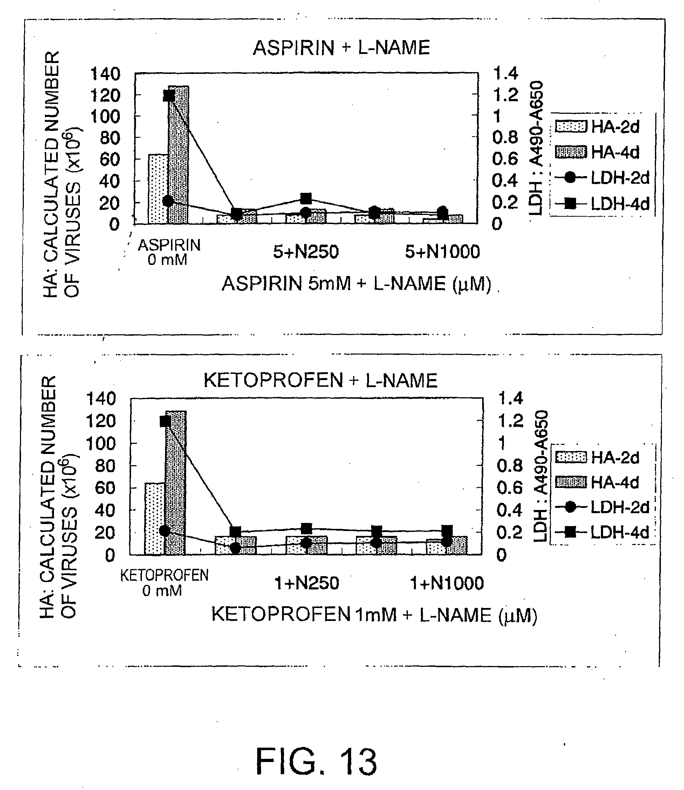

Fig. 13 shows HA activity and the amount of LDH release in the recovered media. LLC-MK2 cells were infected with SeV18+SEAP/ΔF-GFP at MOI=3, two days after infection 5 mM aspirin or 1 mM ketoprofen was added to the media, followed immediately by addition of L-NAME. HA activity and LDH release were measured in the recovered cultured media every two days.

### Best Mode for Carrying Out the Invention

The present invention is specifically illustrated below with reference to Examples, but it is not to be construed as being limited thereto. Furthermore, the references cited throughout this description are incorporated herein by reference.

### [Example 1] Suppression of SeV transcription and replication by addition of aspirin

Aspirin (acetylsalicylic acid) was first selected as the most typical NSAID. Whether or not aspirin affected multiplication (transcription and replication) of Sendai virus (SeV) was examined. Assessment was performed from the point of expression regulation rather than anti-viral effect. Therefore, aspirin (Wako Pure Chemical Industries, Ltd., Osaka, Japan) was added two days after SeV infection, when the virus had already multiplied to some extent.

LLC-MK2 (monkey kidney-derived cells) were seeded into 6-well plates at a concentration of 5x10⁵ cells/well, and then cultured in MEM (Gibco-BRL, Rockville, MD) media containing 10% fetal bovine serum (FBS: BioWhittaker, Walkersville, MD) at 37°C for 24 hours in a 5% CO₂ incubator. Cells were washed with PBS and infected with F-deleted SeV encoding secretion-type alkaline phosphatase (SEAP)) and green fluorescence protein (GFP) genes (SeV18+SEAP/ΔF-GFP: Fig. 1) at MOI=3. F-deleted SeV was prepared using the method described by Li. *et al* (Li, H. -O. *et al*., J. Virology 74, 6564-6569 (2000), WO 00/70070). After infection, cell culture was continued in MEM media without serum. Two days after infection, the media was replaced with MEM media (without serum) containing 0.625, 1.25, 2.5, and 5 mM of aspirin, or no aspirin. Media from each culture was collected and subsequently replaced with fresh media containing the same content every two days. Heme agglutination activity (HA activity) was measured as an indicator of virion formation; lactose dehydrogenase (LDH) quantification was measured as an indicator of cell damage; and SEAP activity was measured as an indicator of the expression level of genes carried on the vector.

GFP expression level and cell damage were also periodically observed using fluorescence microscopy.

HA activity was measured using the method described by Kato *et al*., (Kato, A. *et al*., Genes Cells 1, 569-579 (1996)). That is, virus-containing media was subjected to two-fold serial dilution in a 96 round bottom well plate, such that each well contained 50 ul samples. A 50 ul solution of preserved chicken blood (COSMO BIO Co., Ltd., Tokyo, Japan), diluted to 1% with PBS, was added to each well and mixed. The mixture was incubated at 4°C for one hour. Agglutination of red blood cells was observed, and HA activity was calculated by selecting the highest virus dilution in which agglutination was still observed. Additionally, 1 HA U was considered to be equal to 1x 10⁶ viruses, and values were shown as a number of viruses. The addition of 5 mM aspirin suppressed the increase in HA activity, indicated by a decrease in virion formation (Fig. 2 (A)). LDH quantification was conducted using a Cytotoxicity Detection Kit (Roche, Basel, Switzerland) according to the methodin the kit. Aspirin concentration-dependent decrease in LDH release was observed. Almost no cell damage was observed in cell cultures, especially those containing 5 mM aspirin (Fig. 2(B)). SEAP activities were measured using a Reporter Assay Kit-SEAP (TOYOBO, Osaka, Japan) according to kit method. Aspirin concentration-dependent decrease in SEAP activity was also observed. The most significant decrease in activity was observed in cultures containing 5 mM aspirin (Fig. 2 (C)).

Fluorescence microscopy pictures taken four and six days after aspirin addition (six and eight days after SeV infection, respectively) are shown in Fig. 3. Aspirin concentration-dependent decreases in GFP expression and in cell damage were confirmed. The effect of adding 5 mM aspirin was significant.

Viral proteins were semi-quantitatively analyzed using Western blotting in order to evaluate the transcription and replication levels from another perspective. The same samples as described above were analyzed. That is, LLC-MK2 cells were infected with SeV18+SEAP/ΔF-GFP at MOI=3, aspirin was added two days after infection, the culture media was collected every two days after aspirin addition, and cells were collected six days after aspirin addition (eight days after infection). The culture media was centrifuged at 48,000g for 45 minutes to recover viral proteins, and cells were directly recovered using a cell scraper. Viral proteins were detected by SDS-PAGE followed by Western blotting using anti-M antibody and DN-1 antibody which recognized mainly NP protein (both were rabbit polyclonal antibodies). Western blotting was performed as follows: Cells (stored at -80°C) collected from one well of a 6-well plate, and viral proteins prepared from cultured media, were dissolved in 100 ul SDS-PAGE sample buffer diluted to 1x (Red Loading Buffer Pack; New England Biolabs, Beverly, MA). Samples were heat-treated at 98°C for ten minutes. After centrifugation, 10 ul of supernatant was loaded onto an SDS-PAGE gel (Multigel 10/20; Daiichi Pure Chemicals Co., Ltd, Tokyo, Japan) and electrophoresis was conducted at 15 mA for 2.5 hours. Viral proteins were transferred onto a PVDF membrane (Immobilon PVDF transfer membrane; Millipore, Bedford, MA) by Semi-dry method at 100 mA for one hour. Transfer membranes were then blocked with a blocking solution (Block Ace; Snow Brand Milk Products Co., Ltd, Sapporo, Japan) for one hour at 4°C. Hybridization was performed overnight at 4°C using a primary antibody solution which contained 10% Block Ace and a 1:4,000 dilution of anti-M antibody, or a 1:1,000 dilution of DN-1 antibody. The membranes were washed three times with TBS containing 0.05% Tween 20 (TBST), washed three times with TBS, and then hybridized with secondary antibody solution containing 10% Block Ace and a 1:5,000 dilution of HRP conjugated anti-rabbit IgG antibody (Anti-rabbit IgG (Goat) H+L conj.; ICN P., Aurora, OH) for one hour at room temperature. The membranes were washed with TBST three times, and then washed with TBS three times, and then proteins were detected using chemiluminescence method (ECL Western blotting detection reagents; Amersham Pharmacia Biotech, Uppsala, Sweden). Results showed that viral proteins in the cultured media and in infected cells were both reduced (Fig. 4). Additionally, results of semi-quantitative analysis (Fig. 5) using anti-M antibody correlated well with HA activity (Fig. 2 (A)). Therefore, it was concluded that aspirin addition did indeed suppress SeV transcription and replication.

### [Example 2] Effect of the length of aspirin treatment on the suppression activity of SeV transcription and replication

Differences in viral multiplication were examined for various lengths of aspirin treatment to analyze whether or not suppression of SeV transcription and replication was affected by the length of aspirin treatment, that is, to analyze whether the effect of aspirin addition was permanent or transient.

The experiment was conducted as described in Example 1. That is, LLC-MK2 was plated in a 6-well plate at a concentration of 5x10⁵ cells/well, and incubated in MEM media containing 10% fetal bovine serum at 37°C for 24 hours in a 5% CO₂ incubator. Cells were washed with PBS and infected with SeV18+SEAP/ΔF-GFP at MOI=3. After infection, culture was continued in MEM media without serum. Two days after infection, MEM media (without serum) containing 5 mM aspirin was added. Cultured media was recovered every two days, and at this time fresh media, either with or without 5 mM aspirin, was added. The cultures were treated with aspirin for either 0, 2, 4, 6, or 8 days, and then examined. The recovered culture media was analyzed for HA activity, LDH quantification, and SEAP activity. GFP expression and cell damage were also periodically examined using fluorescence microscopy.

Changes in HA activity (Fig. 6) indicated that the increase in HA activity was low during aspirin treatment, that is, that virion formation was suppressed. However, increases in HA activity and virion formation were observed two days after aspirin treatment had finished. The important finding was that this phenomenon was observed regardless of the length of aspirin treatment, indicating that the effect caused by aspirin addition was transient. LDH responded two days later than HA, increasing four days after aspirin treatment was completed. Thus, LDH release level demonstrated the same phenomenon as HA activity, indicating that the increase was observed regardless of the length of aspirin treatment (Fig. 7). The delay of two days in LDH compared to HA response was thought to reflect a time-lag between the increase in viral transcription and replication (HA activity increase) and LDH release into the media due to cell damage. The sensitive changes observed in HA activity and LDH level were not observed for SEAP activity in response to the commencement and conclusion of aspirin treatment. However, following an initial drop in SEAP activity after aspirin treatment, recovery tended to take about six days (from the conclusion of treatment)(Fig. 8).

Fluorescence microscope images are shown in Fig. 9. These results support the above-described changes in HA activity, LDH level and SEAP activity: During aspirin treatment, GFP expression is low and cell damage is not observed, however, on completion of aspirin treatment, increases in GFP expression and cell damage are observed. These changes were observed regardless of the length of aspirin treatment. It was thus determined that the activity of aspirin in suppressing SeV transcription and replication is transient.

### [Example 3] Effect of NSAIDs other than aspirin

Agents other than aspirin were examined for their activity in suppressing SeV transcription and replication. The agents evaluated were Ketoprofen (Wako Pure Chemical Industries, Ltd., Osaka, Japan), Flurbiprofen (Wako Pure Chemical Industries, Ltd., Osaka, Japan), Indomethacin (Wako Pure Chemical Industries, Ltd., Osaka, Japan), Diclofenac sodium (Wako Pure Chemical Industries, Ltd., Osaka, Japan), Mefenamic acid (Wako Pure Chemical Industries, Ltd., Osaka, Japan), and Papaverin, which acts in smooth muscle relaxation (Wako Pure Chemical Industries, Ltd., Osaka, Japan). Papaverin is not included as an NSAID, but has been reported to suppress Cytomegarovirus multiplication (Albrecht, T. *et al*., Proc Soc Exp Biol Med 186, 41-46 (1987)).

The experiment was conducted as described in Example 1: LLC-MK2 was plated onto a 6-well plate at a concentration of 5x10⁵ cells/well, and incubated in MEM media containing 10% fetal bovine serum at 37°C for 24 hours in a 5% CO₂ incubator. Cells were washed with PBS and infected at MOI=3 with SeV18+SEAP/ΔF-GFP or F-deleted SeV (SeV18+/ΔF-GFP), which did not encode SEAP. After infection, cells were cultured in MEM media without serum for two days, then cultured in MEM media (without serum) containing agents at the recorded concentrations. Cultured media was collected every two days and replaced with a same volume of fresh media. HA activity analysis and LDH quantitative analysis were performed on recovered culture media. GFP expression and cell damage were also periodically examined using fluorescence microscopy.

These results indicated that all agents tested suppressed the expression of genes carried on SeV. The agents tested were generally divided into three groups: (1) Ketoprofen, which suppressed SeV transcription and replication, and reduced cell damage, in the same manner as aspirin; (2) Flurbiprofen and indomethacin, which suppressed SeV transcription and replication, but only moderately reduced cell damage at the concentration tested; and (3) Diclofenac sodium, Mefenamic acid, and Papaverin which suppressed SeV transcription and replication, and enhanced cell damage (Fig. 10 to 11). These results indicated that the suppression of SeV transcription and replication was achieved by many NSAIDs, although the mechanisms of action may be different. Aspirin and ketoprofen, which suppressed SeV transcription and replication as well as reducing cytotoxicity, were thought to possess the most effective characteristics in terms of regulating the expression of genes carried on SeV. Other agents could eventually reduce expression and hence it was revealed that they too, similar to aspirin and ketoprofen, could be used to regulate the expression of genes carried on the vector.

### [Example 4] Investigating the action mechanism

The anti-virus effect of NO is indicated to be the action mechanism by which aspirin suppresses VSV multiplication (Chen, N. *et al*., Virology 276, 44-51 (2000)). Namely, it is thought that suppression of prostaglandin E2 production due to the inhibition of Cyclooxygenase (Cox) activity, which is an acting point of NSAIDs, results in activation of NO synthase (NOS), which in turn leads to enhanced NO production and suppression of VSV transcription and replication. NOS involvement is also suggested in the suppression of VSV transcription and replication by Interleukin-12 (Komatsu, T. *et al*., Virology 259, 334-341 (1999)). Therefore, NO involvement in the suppression of SeV transcription and replication by NSAIDs was examined.

NO's direct effect was examined to determine whether or not addition of NO-associated agents would suppress SeV transcription and replication. However, the addition of isosorbide dinitrate (ISDN: Sigma, St. Louis, MO) or nicorandil (Chugai Pharmaceutical Co., Ltd., Tokyo, Japan) did not significantly suppress transcription and replication (Fig. 12). Therefore, NO was not observed to directly influence suppression. Furthermore, recovery from suppression was not observed when NOS inhibitor L-NAME (DOJINDO LABORATORIES, Kumamoto, Japan) was added to actual SeV transcription and replication suppression conditions, i.e., where 5 mM aspirin or 1 mM ketoprofen had been added (Fig. 13). Thus, the above-described involvement of NO in transcription and replication suppression was not observed in the Examples of the present invention, indicating the involvement of a different mechanism.

### Industrial applicability

The present invention provides a new use for non-steroidal anti-inflammatory drugs and muscle relaxants. These agents can regulate the expression of genes carried on viral vectors, or suppress the cytotoxicity of viral vectors. The present invention is especially useful in regulating the expression of therapeutic and viral genes introduced through viral vectors in gene therapy.

## Claims

1. A method for suppressing the expression of a gene carried on a viral vector, including the step of contacting a non-steroidal anti-inflammatory drug and/or a muscle relaxant with cells transfected by the viral vector.

2. The method according to claim 1, in which cytotoxicity of the viral vector is reduced.

3. The method according to claim 1, in which the non-steroidal anti-inflammatory drug is selected from the group consisting of aspirin, ketoprofen, fulurbiprofen, indomethacin, diclofenac, and mefenamic acid.

4. The method according to claim 3, in which the non-steroidal anti-inflammatory drug is selected from the group consisting of aspirin, ketoprofen, flurbiprofen, and indomethacin.

5. The method according to claim 4, in which the non-steroidal drug is aspirin and/or ketoprofen.

6. The method according to claim 1, in which the muscle relaxant is papaverin.

7. The method according to any one of claims 1 to 6, in which the viral vector is a minus-strand RNA viral vector.

8. The method according to claim 7, in which the minus-strand RNA virus is Paramyxovirus.

9. The method according to claim 8, in which the Paramyxovirus is Sendai virus.

10. An agent for suppressing the expression of a gene carried on a viral vector, wherein the agent comprises a non-steroidal anti-inflammatory drug and/or a muscle relaxant.

11. The agent according to claim 10, in which the agent has the ability to reduce the cytotoxicity of the viral vector.
